(19) Europäisches Patentamt
European Patent Office
Office européen des brevets

(11) Publication number: 0 005 698 A1

(12) EUROPEAN PATENT APPLICATION

(21) Application number: 78100068.2

(22) Date of filing: 01.06.78

(51) Int. Cl.[2]: C 07 C 179/14
A 01 N 9/26

(43) Date of publication of application: 12.12.79 Bulletin 79/25

(84) Designated Contracting States: BE CH DE FR GB LU NL SE

(71) Applicant: Kalo Laboratories, Inc.
9233 Ward Parkway
Kansas City, Missouri 64114 (US)

(72) Inventor: Nash, Lawrence H.
P.O.Box 23837
Fort Lauderdale, Florida 33307 (US)

(74) Representative: Abitz, Walter, Dr.-Ing. et al,
Abitz, Morf, Gritschneder P.O.Box 860109
D-8000 München 86 (DE)

(54) **Di(halogenated phenoxyalkanoyl)peroxides and their use as herbicides.**

(57) Compounds are disclosed which have the formula:

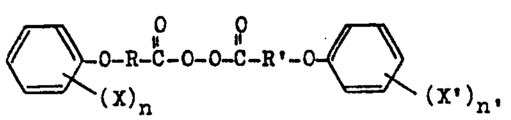

wherein X and X' are each chlorine, R and R' are each alkyl groups containing from 1 to 4 carbon atoms and n and n' each have a value from 1 to 5. The disclosed compounds are useful as herbicides.

EP 0 005 698 A1

Croydon Printing Company Ltd.

TITLE MODIFIED
see front page

TITLE

BIS HALOGENATED PHENOXYALKANOATES AND THEIR USE AS HERBICIDES

TECHNICAL FIELD

The present invention relates to halogenated phenoxyalkanoates useful as plant growth regulators and more particularly to novel bis halogenated phenoxyalkanoates that are effective for achieving complete control, complete extermination and removal of undesired plants at a reasonable cost with the regrowth of plants treated with said novel compounds.

BACKGROUND ART

Halogenated phenoxyalkanoic acids are well known in the art, however, these compounds exhibit undesired harmful effects, when used in the form of emulsions, inverted emulsions, esters, ethers, esters, alkali solutions, amine salts and acetates. In addition said compounds and compositions have been found to be unstable under certain conditions as well as highly toxic to aquatic life.

Halogenated phenoxyalkanoates have also been extensively used as plant growth regulators. One such compound is 2,4-dichlorophenoxyacetic acid. The compound, as well as the alkali metal salts thereof, are slightly soluble in water. These compounds have been found to be highly toxic to aquatic life. A higher analog, the sodium salt of 2,4,5-trichlorophenoxypropionic acid, is not permitted to be used because of its toxicity.

## BRIEF SUMMARY OF THE INVENTION

This invention relates to compounds of the formula:

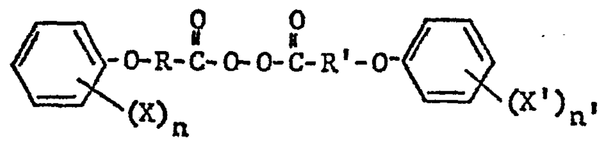

wherein X and X' are halogen, R and R' are alkyl groups containing from 1 to 4 carbon atoms and n and n' have a value from 1 to 5. These compounds are useful as herbicides.

The present invention provides effective plant growth regulators which are effective for achieving complete control, complete extermination and removal of undesired plants at a reasonable cost without harmful effects to aquatic life.

Also provided are water-insolüble plant growth regulators that are free of alkali materials, amines or any other matter that would change the plant growth regulator molecule.

Further, this invention provides novel compounds that can be produced without the formation of side products or impurities that can have harmful effects on the plant growth regulating activities of the novel compounds of this invention.

The novel compounds of this invention exhibit a favorable rate of disappearance from the soil after application thereby avoiding residual action by remaining in the soil after the peak desired period for chemical control has passed.

The compounds of this invention are useful as general plant growth regulators on lawns, golf courses, parks, playgrounds, home gardens, farms, ditches, canals, ponds, lakes, nurseries and whereever the control of plants are necessary. Consistent with this effect, the compounds of this invention have no ill effect upon aquatic life nor on water so treated, especially when used for

irrigation, human or animal purposes.

Finally, the new plant growth regulators of this invention can be made readily available at reasonable prices and can be incorporated into a variety of compositions thereby facilitating their application and use.

This invention will be better understood and its advantageous effects other than those set forth above will become apparent when consideration is given to the following detailed description of the invention including the various embodiments thereof.

DETAILED DESCRIPTION OF THE INVENTION
AND STATEMENT OF INDUSTRIAL APPLICATION

The present invention relates to compounds of the formula:

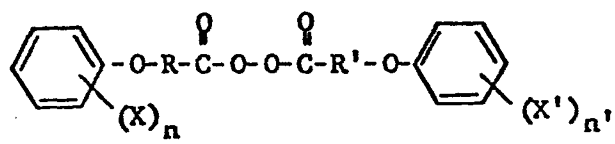

wherein X and X' are halogen, R and R' are alkyl groups containing from 1 to 4 carbon atoms and n and n' have a value from 1 to 5.

More specifically, X and X' are both halogen with such halogen generally being chlorine. Both R and R' can be the same or different alkyl groups. Each such alkyl group may contain from 1 to 4 carbon atoms, and preferably contains from 1 to 2 carbon atoms. The value of n and n' may each generally be from 1 to 5 inclusive and preferably each has a value of 2.

The preferred compounds of this invention are bis 2,4-dichlorophenoxyacetate and bis 2,4-dichlorophenoxypropionate.

The compounds of the invention are useful as plant growth regulators and are preferably employed in herbicidal amounts. Some of the plants controlled by the novel compounds of this invention include, for example, hydrilla, coontail,

watermillfoil, pondweed, stinkweed, naiad, bullrush, chickweed, cockleburr, duckweed, elderberry, morning glory, mustard, waterlilly, dogfennel, pigweed and many other common weeds.

The compounds of this invention are water-insoluble and are therefore capable of removing themselves from soil and water by means of decomposition due to soil bacteria and have been found to be non-toxic to aquatic life and small animals, yet the compounds can be made water-miscible, if so desired.

A method for preparing the compounds of the present invention comprises the reaction of: (a) 2 moles of at least one compound of the formula:

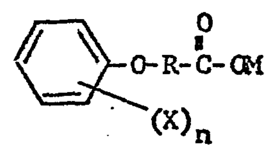

wherein M is ammonium or an alkali metal and R is an alkyl containing from 1 to 4 carbon atoms, and (b) 1 mole of a compound of the formula:

$$M'_2S_2O_8.$$

wherein M' is ammonium or an alkali metal.

The novel compounds can be prepared by using compound (a) as a reactant or by initially forming said compound (a) by the reaction of two moles of a halogenated, preferably chlorinated, phenoxyalkanoic acid with an equimolar amount of an aqueous solution of an alkali metal or ammonium hydroxide which is heated to a temperature sufficient to cause said halogenated phenoxyalkanoic acid to become solubilized therein just prior to the addition of said acid. The resulting reaction product which is present in solution contains a compound corresponding to compound (a), above, two moles of said compound then being reacted with an aqueous solution of 1 mole of a compound (b) wherein M is ammonium or an alkali metal, said alkali metal generally being sodium or potassium. Compound (b) is preferably

ammonium persulfate and is preferably present in 1,000 ml. of water when combined with compound (a) to form the novel compounds of this invention.

In accordance with a preferred embodiment of this invention, 2 moles of 2,4-dichlorophenoxyacetic acid is initially added to a heated aqueous solution of potassium hydroxide (wherein 2 moles of potassium hydroxide is present in 1,000 ml. of water). The aqueous solution of potassium hydroxide is then heated to a temperature sufficient to cause said 2,4-dichlorophenoxyacetic acid to become soluble in said water which is generally achieved at temperatures up to about 95°C. A clear light brown solution results and 1 mole of ammonium persulfate in 1,000 ml. of water is then preferably added to the light brown solution resulting in the formation of a light tan precipitate that is recovered by filtration and suction.

The tan precipitate, bis 2,4-dichlorophenoxyacetate, is preferably dried, under vacuum at a pressure of about 5 mm. pressure and at a temperature of between about 70 and 90°C and preferably between 80 and 90°C with best results at 80°C. The filtrate contains 1 mole of ammonium sulfate and 1 mole of potassium sulfate that can be recovered by drying and can be subsequently used as an agricultural fertilizer.

In preparing the compounds of this invention, other halogenated phenoxyalkanoic acids can be substituted for the 2,4-dichlorophenoxyacetic acid, including, for example, p-chlorophenoxyacetic acid, 2,4,5-trichlorophenoxypropionic acid, 2,3,4,5,6-pentachlorophenoxypropionic acid, 2,3,5,6-tetrachlorophenoxyacetic acid, forming respectively, bis p-chlorophenoxyacetate, bis 2,4,5-trichlorophenoxypropionate, bis 2,3,4,5,6-pentachlorophenoxypropionate and bis 2,3,5,6-tetrachlorophenoxyacetate.

The compounds of the present invention can be employed in combination with an inert carrier. To make such a combination, the compounds of the present invention can be uniformly admixed with any of the well known free-flowing particulate dry inert solid carriers which may be organic or inorganic. These inert carriers include, for example, sawdust, the flour derived from soybeans, tobacco, walnut shell, wheat, wood by-products, lignin and lignocellulose, ligninsulfonic acid, cork, urea-formaldehyde, resins, silicas, carbonates, calcite, dolomite, silicates, tricalcium phosphate, boric acid, etc.

The amount of the active compound of the invention which should be combined with a carrier as above described is determined by the intended use of the composition. In general, it is preferable to employ an herbicidal amount of the compound of the invention.

In addition, the compositions may optionally contain from about .5 to about 1.0 weight percent of a surfactant or wetting agent, which renders the products wettable and dispersable, thereby facilitating the application thereof in the field.

The ingredients may be simply mixed together thoroughly with said inert carrier or blended and then passed through a highspeed grinder, after which the mixture is a free-flowing product.

The surfactants disclosed in Bulletin E-607 of the Bureau of Entomology and Plant Quarantine of the United States Department of Agriculture, or surface active agents disclosed in U.S. Patents, Nos. 2,426,417; 2,655,447; 2,412,510 and 2,139,276, are suitable for use in the practice of this invention.

Examples of suitable formulations include the following:

EXAMPLE ONE

Three pounds (1.36 kg.) of a composition in the form of a 325 mesh powder and containing the following ingredients:

| | By weight: |
|---|---|
| Bis 2,4-dichlorophenoxyacetate | 40% |
| Surfactant (Dupanol C) | 1% |
| Diatomaceous earth (Attaclay) | 59% |

was added to 100 gallons (378.53 l.) of water. This aqueous composition will control the following weeds present with sugarcane: spiny amaranth, carpetweed, geranium, cressleafed groundsel, common lambsquarter, mexicantea, pellitoryweed, common purslane, ragweed, sowthistel and toadflax.

EXAMPLE TWO

Field corn was planted in 3 foot (0.91 m.) square flats wherein the seeds were equally spaced at 4 inch (10.16 cm.) intervals. After planting, the surface of each flat was dusted with 9.4 gms. of a powder composition containing the following:

| | By weight: |
|---|---|
| Bis 2,4-dichlorophenoxyacetate | 10% |
| Surfactant (Dupanol C) | 1% |
| Diatomaceous earth (Attaclay) | 89% |

The ingredients were initially mixed in a ribbon type blender and are thereafter passed through a high speed grinder to form a smooth, 325 mesh powder.

The corn germination was ninety-two percent on three replicated flats while there were no broad leaf weeds observed in the flats.

EXAMPLE THREE

Thirty pounds (13.61 kg.) of bis 2,4-dichlorophenoxyacetate was dusted on one acre (0.40 ha.) of a canal heavily infested with underwater growth. Hydrilla, coontail, watermillfoil, waterlilly, pondweed and naiad were each observed as being successfully controlled at the end of a three-week period. No ill effect upon aquatic life was observed.

EXAMPLE FOUR

A liquid composition was prepared by dissolving 2,722 gms., of bis 2,4-dichlorophenoxyacetate in 1,000 gms. of a 50 percent solution of ethylenediamine in water. Forty grams of surfactant, amine salt of a higher secondary alkyl sulfate, was added thereto, with agitation. Water was added to make one gallon (3.78 l.). One quart (0.95 l.) of this composition, when added to 100 gallons (378.53 l.) of water and sprayed upon an acre (0.40 ha.) of uncultivated raw land, controls the following weeds; bindweed, bullthistle, chickweed, cockleburr, dogfennel, duckweed, elderberry, morning glory, mustard, pigweed, purslane, sowthistle, spiny amaranth, stinkweed, thistles, virginia creeper and wild garlic.

EXAMPLE FIVE

Two moles of 2,4-dichlorophenoxyacetic acid were dissolved in 1,000 ml. of water containing 2 moles of potassium hydroxide. One mole of ammonium persulfate was then dissolved in another 1,000 ml. of water and then the 2 aqueous solutions were admixed with each other. Instead of filtering the precipitated bis 2,4-dichlorophenoxyacetate, the total reaction product was vacuum dried within the temperature range of 70 to 90°C and at about 5 mm. Hg of pressure. The yield was 725 gms. which is 97% of the theoretical yield. The anhydrous product produced consists of bis 2,4-dichlorophenoxyacetate, ammonium sulfate and potassium sulfate, said homogenous mixture thereafter being passed through a high speed grinder to produce a free flowing powder.

EXAMPLE SIX

The homogenous mixture as prepared in Example Five is added to 6,525 gms. of talc and ground in a ball mill for eight hours resulting in the formation of a fine free-flowing powder. This powder was used to cover the surface of the furrow when corn was being cultivated. The powder covered about an acre and a half (0.61 ha.). Unexpectedly, the weeds in this treated area were controlled and the small amount of fertilizer was beneficial to the corn.

0005698

## CLAIMS

1. A compound having the formula:

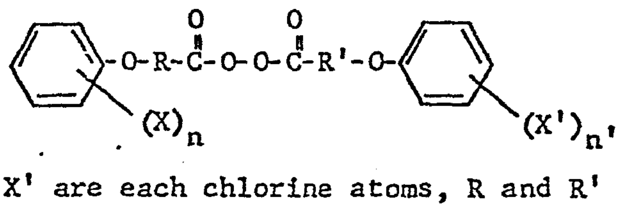

wherein X and X' are each chlorine atoms, R and R' are each alkyl groups containing from 1 to 4 carbon atoms and n and n' each have a value from 1 to 5.

2. The use of the compound of claim 1 as an herbicide.

3. The compound of claim 1 wherein R and R' are each methyl or ethyl.

4. The use of the compound of claim 3 as an herbicide.

5. The compound of claim 1 wherein R and R' are each methyl and n and n' each have a value of 2.

6. The use of the compound of claim 5 as an herbicide.

7. The compound of claim 1 wherein R and R' are each ethyl and n and n' each have a value of 2.

8. The use of the compound of claim 7 as an herbicide.

European Patent Office

# EUROPEAN SEARCH REPORT

0005698
Application number

EP 78 10 0068

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| A | GB - A - 1 086 405 (WALLACE & TIERNAN) * Claim 1 * -- | | C 07 C 179/14<br>A 01 N 9/26 |
| A | DE - A - 1 957 386 (LILJE HOLMES STEARINFABRIKS) * Claim 1 * ---- | | |

TECHNICAL FIELDS SEARCHED (Int.Cl.²)

C 07 C 179/14
A 01 N 9/26

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 21-08-1978 | Mme BONNEVALLE |

EPO Form 1503.1 06.78